# EUROPEAN PATENT SPECIFICATION

(11) **EP 4 046 604 B1**
(45) Date of publication and mention of the grant of the patent: **22.07.2026**
(21) Application number: 22156719.1
(22) Date of filing: 15.02.2022
(51) Int. Cl.: A61F 2/24, A61F 2/966, A61F 2/95

(54) **TRANSCATHETER VALVE REPLACEMENT DELIVERY DEVICE WITH ENGAGEABLE CAPSULE PORTIONS**
TRANSKATHETER-KLAPPENERSATZEINFÜHRVORRICHTUNG MIT EINRASTBAREN KAPSELTEILEN
DISPOSITIF D'ADMINISTRATION DE REMPLACEMENT DE VALVULE TRANSCATHÉTER AVEC DES PARTIES DE CAPSULE POUVANT VENIR EN PRISE

(30) Priority: 22.02.2021 US 202163152161 P; 03.02.2022 US 202217592185
(43) Date of publication of application: 24.08.2022
(73) Proprietor: Medtronic, Inc., Minneapolis, MN 55432 (US)
(72) Inventor: SANDQUIST, Kelsey M., Santa Rosa (US); GROSSMAN, David A., Santa Rosa (US); GRIMM, Emily A., Santa Rosa (US); DAVE, Chitrang M., Santa Rosa (US)
(74) Representative: Zimmermann & Partner Patentanwälte mbB

(56) References cited:
- US-A1- 2010 100 167
- US-A1- 2019 209 310
- US-B2- 10 149 758

## Description

### CROSS-REFERENCE TO RELATED APPLICATIONS

This Non-Provisional Patent Application claims the benefit of the filing date of U.S. Provisional Patent Application Serial Number 63/152,161, filed February 22, 2021, entitled "TRANSCATHETER VALVE REPLACEMENT DELIVERY DEVICE WITH ENGAGEABLE CAPSULE PORTIONS AND METHODS,".

### FIELD

The present technology is generally related to transcatheter valve replacement delivery devices and methods of transcatheter valve repair. Various aspects of the disclosure are particularly beneficial for tricuspid valve repair.

### BACKGROUND

A human heart includes four heart valves that determine the pathway of blood flow through the heart: the mitral valve, the tricuspid valve, the aortic valve, and the pulmonary valve. The mitral and tricuspid valves are atrio-ventricular valves, which are between the atria and the ventricles, while the aortic and pulmonary valves are semilunar valves, which are in the arteries leaving the heart. Ideally, native leaflets of a heart valve move apart from each other when the valve is in an open position, and meet or "coapt" when the valve is in a closed position. Problems that may develop with valves include stenosis in which a valve does not open properly, and/or insufficiency or regurgitation in which a valve does not close properly. Stenosis and insufficiency may occur concomitantly in the same valve. The effects of valvular dysfunction vary, with regurgitation or backflow typically having relatively severe physiological consequences to the patient.

Diseased or otherwise deficient heart valves can be repaired or replaced using a variety of different types of heart valve surgeries. One conventional technique involves an open-heart surgical approach that is conducted under general anesthesia, during which the heart is stopped and blood flow is controlled by a heart-lung bypass machine.
US 2019/209310 A1 describes a prosthetic heart valve and a delivery apparatus. The delivery apparatus may include a main catheter 102 having an outer shaft 104 and a nose-cone catheter 118 having a nose piece or nose cone 122. Outer shaft 104 can comprise a distal segment 126 that comprises a slotted metal tube. In an alternative embodiment a sheath 1000 may be provided which has a construction similar to sheath 106 except that the sheath 1000 has a plurality of circumferentially spaced flexible flaps 1002 at its distal end.

More recently, minimally invasive approaches have been developed to facilitate catheter-based implantation of a prosthetic heart valve or prosthesis on the beating heart, intending to obviate the need for the use of classical sternotomy and cardiopulmonary bypass. In general terms, an expandable prosthetic valve is compressed about or within a catheter, inserted inside a body lumen of the patient, such as the femoral artery, and delivered to a desired location in the heart.

The heart valve prosthesis employed with catheter-based, or transcatheter, procedures generally includes an expandable multi-level frame or stent that supports a valve structure having a plurality of leaflets. The frame can be contracted during percutaneous transluminal delivery, and expanded upon deployment at or within the native valve. One type of valve stent can be initially provided in an expanded or uncrimped condition, then crimped or compressed about a balloon portion of a catheter. The balloon is subsequently inflated to expand and deploy the prosthetic heart valve. With other stented prosthetic heart valve designs, the stent frame is formed to be self-expanding. With these systems, the valved stent is crimped down to a desired size and held in that compressed state within a sheath for transluminal delivery. Retracting the sheath from this valved stent allows the stent to self-expand to a larger diameter, fixating at the native valve site. In more general terms, then, once the prosthetic valve is positioned at the treatment site, for instance within an incompetent native valve, the stent frame structure may be expanded to hold the prosthetic valve firmly in place.

The present disclosure addresses problems and limitations associated with the related art.

### SUMMARY

The techniques of this disclosure generally relate to delivery devices and methods for transcatheter delivery and deployment of a prosthesis, such as a prosthetic heart valve, to a defective heart valve. Aspects of the disclosure are particularly beneficial for transcatheter tricuspid valve repair as various delivery devices are configured to reduce the depth in which the device needs to be inserted into the right ventricle during delivery of the prosthesis. Access to a tricuspid valve can be challenging in that existing implanted devices may be in the anatomy, reducing the space available for the delivery device. In addition, visualization of the delivery system and implant may be challenging as metallic capsules can cause artifacts due to density. Further, chordae, papillary muscles serve as obstacles for delivery and the right ventricle is generally shorter than the left ventricle. All of these considerations result in a general desire for a system capable of delivering an implant to a tricuspid valve while reducing a length the delivery device extends into the right ventricle and past the valve annulus.

Aspects of the disclosure provide a delivery device including a handle assembly as well as an inner catheter connected to the handle assembly. The inner catheter has a distal portion configured to receive an implant. The delivery device further includes an outer catheter connected to the handle assembly and coaxially positioned over the inner catheter. The delivery device also includes a capsule assembly including a proximal capsule secured to a distal end of the inner catheter. The capsule assembly also includes a distal capsule connected to a capsule shaft that extends within the inner catheter, the capsule shaft interconnecting the distal capsule to the handle assembly. The capsule assembly includes engagement features to releasably secure the distal capsule to the proximal capsule. Methods of loading and releasing an implant from a delivery device are also disclosed.

In one aspect, the present disclosure includes a delivery device including a capsule assembly having a proximal capsule and a distal capsule. The proximal capsule and the distal capsule may be engaged with one or more engagement features in a loaded arrangement to sheath an implant. The engagement features can be selectively disengaged to at least partially unsheathe the implant.

In one aspect, the present disclosure provides a delivery device including a handle assembly as well as an inner catheter connected to the handle assembly. The inner catheter has a distal portion configured to receive an implant. The delivery device further includes an outer catheter connected to the handle assembly and coaxially positioned over the inner catheter. The delivery device also includes a capsule assembly including a proximal capsule secured to a distal end of the inner catheter. The proximal capsule defining a plurality of slats separated by a plurality of slits. The capsule assembly also includes a distal capsule connected to a capsule shaft that extends within the inner catheter, the capsule shaft interconnecting the distal capsule to the handle assembly. The capsule assembly includes engagement features to releasably secure the distal capsule to the proximal capsule.

In another aspect, the disclosure provides methods of releasing an implant from a delivery device. Such methods can include providing a delivery device in a loaded arrangement having an implant loaded within a capsule assembly. The capsule assembly including a proximal capsule and a distal capsule. The distal portion and the proximal portion are engaged in the loaded arrangement with a plurality of corresponding engagement features. The method further includes disengaging the distal capsule from the proximal capsule.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1A is a top isometric view of a prosthetic heart valve in an expanded arrangement.
FIG. 1B is a cross-sectional side view of the prosthetic heart valve of FIG. 1A.
FIG. 1C is a top view schematically illustrating the prosthetic heart valve of FIGS. 1A-1B.
FIG. 2A is a perspective view of a delivery device suitable for delivering the prosthetic heart valve of FIGS. 1A-1C.
FIG. 2B is a partial, exploded view of the delivery device of FIG. 2A.
FIG. 3 is a schematic cross-sectional view of select components of the delivery device of FIGS. 2A-2B.
FIG. 4 is a partial, perspective view of a distal end of the delivery device of FIGS. 2A-3 having the implant of FIGS. 1A-1C partially compressed within a capsule assembly.
FIG. 5A is a schematic, cross-sectional view of the capsule assembly of FIG. 4 in a loaded arrangement.
FIG. 5B is a cross-sectional view of the capsule assembly of FIGS. 4-5A in a partially-deployed arrangement.
FIG. 5C is a schematic, cross-sectional view of the capsule assembly of FIG. 4 in a loaded arrangement.
FIGS. 6A-6B are partial, schematic illustrations of an alternate capsule assembly in a loaded arrangement, the capsule assembly including a distal capsule and proximal capsule having corresponding engagement features.
FIGS. 6C-6D are partial, schematic illustrations of the capsule assembly of FIGS. 6A-6B in a partially-deployed arrangement.
FIG. 7 is a partial, schematic illustration of the capsule assembly of FIGS. 6A-6D.
FIG. 8 is a schematic illustration of the proximal capsule of the capsule assembly of FIGS. 6A-7.
FIG. 9 is a partial, enlarged view of the engagement between the proximal capsule and the distal capsule of the capsule assembly of FIGS. 6A-8.
FIG. 10 is a schematic illustration of an alternate capsule assembly having corresponding engagement features.
FIG. 11 is an enlarged, schematic illustration of two of the engagement features of FIG. 10.
FIG. 12A is a partial, cross-sectional view of an alternate capsule assembly having corresponding engagement features in an engaged arrangement.
FIG. 12B is a partial, cross-sectional view of the capsule assembly of FIG. 12A having the corresponding engagement features in a disengaged arrangement.
FIG. 13 is a partial, cross-sectional view of another capsule assembly having corresponding engagement features in an engaged arrangement.
FIG. 14 is a partial, cross-sectional view of another capsule assembly having corresponding engagement features in an engaged arrangement.
FIG. 15 is a partial, cross-sectional view of another capsule assembly having corresponding engagement features in an engaged arrangement.
FIG. 16 is a partial, cross-sectional view of another capsule assembly having corresponding engagement features in a disengaged arrangement.
FIG. 17 is a partial, cross-sectional view of another capsule assembly having corresponding engagement features in a disengaged arrangement.
FIG. 18A is a partial, schematic illustration of an alternate capsule assembly having a proximal capsule and distal capsule that are sewn together.
FIG. 18B is an enlarged, schematic illustration of the capsule assembly of FIG. 18A.
FIG. 19 is a partial, cross-sectional view of another capsule assembly having corresponding engagement features in an engaged arrangement.
FIG. 20 is a partial, cross-sectional view of another capsule assembly having corresponding engagement features in an engaged arrangement.
FIGS. 21-23 collectively schematically illustrate an alternate capsule assembly deployable with a balloon.
FIG. 24 is a partial, schematic illustration of a capsule assembly in the loaded arrangement, fully sheathing the implant.
FIG. 25 is a partial, schematic illustration of a method of delivering and deploying the implant to a tricuspid valve.

### DETAILED DESCRIPTION

Specific embodiments of the present disclosure are now described with reference to the figures, wherein like reference numbers indicate identical or functionally similar elements. The terms "distal" and "proximal" are used in the following description with respect to a position or direction relative to the treating clinician. "Distal" or "distally" are a position distant from or in a direction away from the clinician. "Proximal" and "proximally" are a position near or in a direction toward the clinician.

As referred to herein, implants, stented prostheses, stented prosthetic heart valves or "prosthetic valves" useful with the various systems, devices and methods of the present disclosure may assume a wide variety of configurations. Stented prosthetic heart valves can include, for example, a bioprosthetic heart valve having tissue leaflets or a synthetic heart valve having polymeric, metallic or tissue-engineered leaflets, and can be specifically configured for replacing valves of the human heart. The prosthetic valves and stented prostheses of the present disclosure may be self-expandable, balloon expandable and/or mechanically expandable or combinations thereof. In general terms, the prosthetic valves of the present disclosure include a stent or stent frame having an internal lumen maintaining a valve structure (tissue or synthetic), with the stent frame having an uncompressed, expanded condition or arrangement and collapsible to a compressed condition or arrangement for loading within the delivery device. For example, the stents or stent frames are support structures that comprise a number of struts or wire segments arranged relative to each other to provide a desired compressibility and strength to the prosthetic valve. The struts or wire segments are arranged such that they are capable of self-transitioning from, or being forced from, a compressed or collapsed arrangement to a normal, radially expanded arrangement. The struts or wire segments can be formed from a shape memory material, such as a nickel titanium alloy (e.g., Nitinol). The stent frame can be laser-cut from a single piece of material, or can be assembled from a number of discrete components.

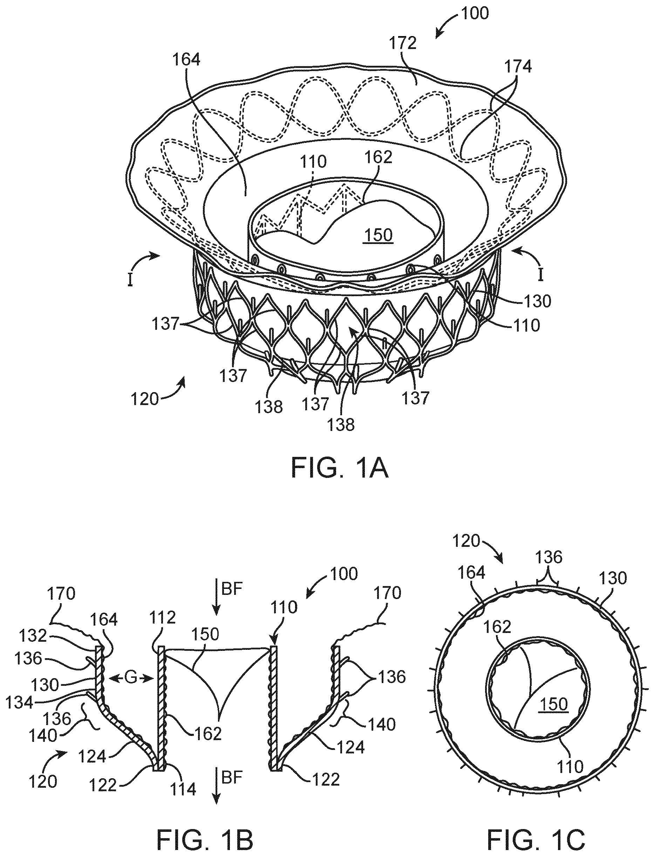

One non-limiting example of an implant, that being a stented prosthetic heart valve 100, is illustrated in FIGS. 1A-1C, The implant 100 includes a valve support 110, an anchoring member 120 attached to the valve support 110, and a prosthetic valve assembly 150 within the valve support 110. Referring in particular to FIG. 1B, the valve support 110 has an inflow region 112 and an outflow region 114. The prosthetic valve assembly 150 is arranged within the valve support 110 to allow blood to flow from the inflow region 112 through the outflow region 114 (arrows BF), but prevent blood from flowing in a direction from the outflow region 114 through the inflow region 112.

The anchoring member 120 includes a base 122 attached to the outflow region 114 of the valve support 110 and a plurality of arms 124 projecting laterally outward from the base 122. The anchoring member 120 also includes a fixation structure 130 extending from the arms 124. The fixation structure 130 can include a first portion 132 and a second portion 134. The first portion 132 of the fixation structure 130, for example, can be an upstream region of the fixation structure 130 that, in a deployed configuration as shown in FIG. 1B, is spaced laterally outward apart from the inflow region 112 of the valve support 110 by a gap G. The second portion 134 of the fixation structure 130 can be a downstream-most portion of the fixation structure 130. The fixation structure 130 can be a cylindrical ring (e.g., straight cylinder or conical), and the outer surface of the fixation structure 130 can define an annular engagement surface configured to press outwardly against the native annulus. The fixation structure 130 can further include a plurality of fixation elements 136 that project radially outward and are inclined toward an upstream direction. The fixation elements 136, for example, can be barbs, hooks, or other elements that are inclined only in the upstream direction (e.g., a direction extending away from the downstream portion of the implant 100).

The anchoring member 120 has a smooth bend 140 between the arms 124 and the fixation structure 130. For example, the second portion 134 of the fixation structure 130 extends from the arms 124 at the smooth bend 140. The arms 124 and the fixation structure 130 can be formed integrally from a continuous strut or support element such that the smooth bend 140 is a bent portion of the continuous strut. In other examples, the smooth bend 140 can be a separate component with respect to either the arms 124 or the fixation structure 130. For example, the smooth bend 140 can be attached to the arms 124 and/or the fixation structure 130 using a weld, adhesive or other technique that forms a smooth connection. The smooth bend 140 is configured such that the implant 100 can be recaptured in a capsule or other container after the implant 100 has been at least partially deployed.

The implant 100 can further include a first sealing member 162 on the valve support 110 and a second sealing member 164 on the anchoring member 120. The first and second sealing members 162, 164 can be made from a flexible material, such as a polymeric material. The first sealing member 162 can cover the interior and/or exterior surfaces of the valve support 110. The first sealing member 162 is attached to the interior surface of the valve support 110, and the prosthetic valve assembly 150 is attached to the first sealing member 162 and commissure portions of the valve support 110. The second sealing member 164 is attached to the inner surface of the anchoring member 120. As a result, the outer annular engagement surface of the fixation structure 130 is not covered by the second sealing member 164 so that the outer annular engagement surface of the fixation structure 130 directly contacts the tissue of the native annulus.

The implant 100 can further include an extension member or brim 170. The extension member 170 can be an extension of the second sealing member 164, or it can be a separate component attached to the second sealing member 164 and/or the first portion 132 of the fixation structure 130. The extension member 170 can be a flexible member that, in a deployed state as shown in FIGS. 1A-1B, flexes relative to the first portion 132 of the fixation structure 130. In operation, the extension member 170 guides the implant 100 during implantation such that the device is located at a desired elevation and centered relative to the native annulus. In some embodiments, one or more components of the extension member 170 can be made of or include a radiopaque material.

As best shown in FIG. 1A, valve support 110 defines a first frame (e.g., an inner frame) and fixation structure 130 of the anchoring member 120 defines a second frame (e.g., an outer frame) that each include a plurality of structural elements. The fixation structure 130, more specifically, includes structural elements 137 arranged in diamond-shaped cells 138 that together form at least a substantially cylindrical ring when freely and fully expanded as shown in FIG. 1A. The structural elements 137 can be struts or other structural features formed from metal, polymers, or other suitable materials that can self-expand or be expanded by a balloon or other type of mechanical expander.

The fixation structure 130 can be a generally cylindrical fixation ring having an outwardly facing engagement surface. For example, in the embodiment shown in FIG. 1A, the outer surfaces of the structural elements 137 define an annular engagement surface configured to press outwardly against the native annulus in the deployed state. In a fully expanded state without any restrictions, the fixation structure 130 is at least substantially parallel to the valve support 110. However, the fixation structure 130 can flex inwardly (arrow I) in the deployed state when it presses radially outwardly against the inner surface of the native annulus of a heart valve.

The first sealing member 162 lines the interior surface of the valve support 110, and the second sealing member 164 along the inner surface of the fixation structure 130. The extension member 170 has a flexible web 172 (e.g., a fabric) and a support member 174 (e.g., metal or polymeric strands) attached to the flexible web 172. The flexible web 172 can extend from the second sealing member 164 without a metal-to-metal connection between the fixation structure 130 and the support member 174. For example, the extension member 170 can be a continuation of the material of the second sealing member 164. Several embodiments of the extension member 170 are thus a floppy structure that can readily flex with respect to the fixation structure 130. The support member 174 can have a variety of configurations and be made from a variety of materials, such as a double-serpentine structure made from Nitinol. Additional details regarding the implant 100 can be found in U.S. Pat. Ser. No. 15/643,011.

In one example, the implant 100 is a prosthetic tricuspid heart valve having a 48 mm compressed outer diameter and a compressed length of 30.5 mm within a capsule having a 29 Fr inner diameter. In yet another example, the implant is a prosthetic tricuspid valve implant having a 54 mm compressed outer diameter and a compressed length of 34.3 mm within a capsule having a 29 Fr inner diameter.

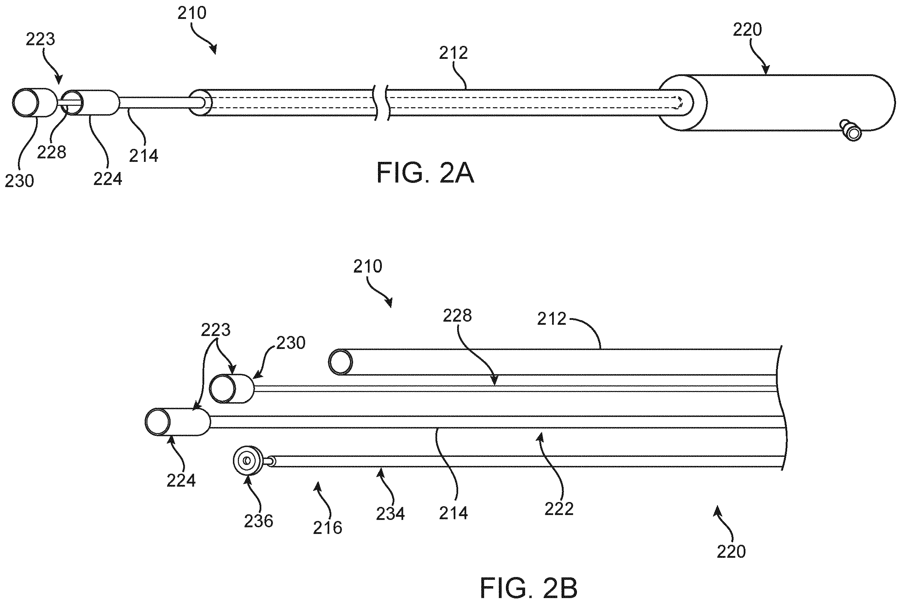

By way of background, a delivery device for transcatheter delivery of an implant of the disclosure, such as implant of FIGS. 1A-1C is collectively shown in FIGS. 2A-5C. In general terms, the delivery device 210 is arranged and configured for percutaneously delivering a stented prosthetic heart valve 100 or other implant or prosthesis to a patient's native defective heart valve. The delivery device 210 includes an optional outer sheath 212, a proximal capsule assembly 214, a valve retainer assembly 216 and a distal capsule assembly 218, all of which are connected to and controlled with a handle assembly 220. The proximal capsule assembly 214 is positioned within the outer sheath 212 and includes a catheter 222 connected to a proximal capsule 224. Optionally, the catheter 222 can be integrally formed with the proximal capsule 224. The distal capsule 230 includes a shaft 228 positioned within the catheter and a distal capsule 230. Optionally, the shaft 228 may be hollow to define a lumen to receive other components of the delivery system, such as guidewire. Collectively, the distal and proximal capsules 224, 230 form a capsule assembly 223 for receiving and compressively retaining an implant (e.g., implant 100). To further support and releasably maintain the implant, the valve retainer assembly 216 includes an inner catheter 234 positioned within the inner catheter 222, which is connected to a piston 236 configured to releasably maintain the implant. The piston 236 can be of any of the type known and used for releasably maintaining an implant within a capsule or sheath for transcatheter delivery.

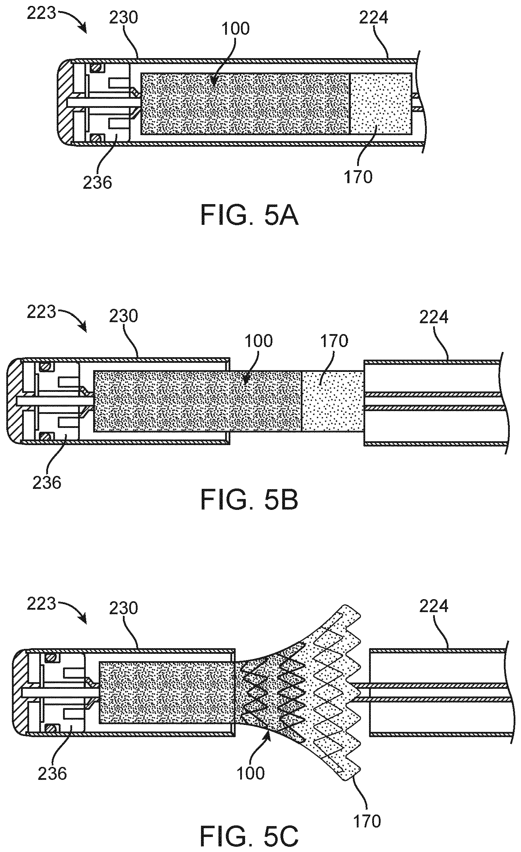

The delivery device 210 provides a loaded, compressed arrangement (FIG. 5A) in which the prosthetic valve or implant 100 is loaded onto the piston 236 and is compressively retained within the capsule assembly 223. The distal capsule 230 and the proximal capsule 224 may be abutting in the compressed, loaded arrangement. Once loaded and compressed, the prosthetic valve 100 is located at a target site, the proximal and distal capsules 224, 230 are separated to allow the implant 100 to naturally expand and release from the piston 236. Once the implant 100 is deployed, the distal capsule 230 can be drawn through the implant 100 and proximally withdrawn with the delivery device 210 for removal from the patient.

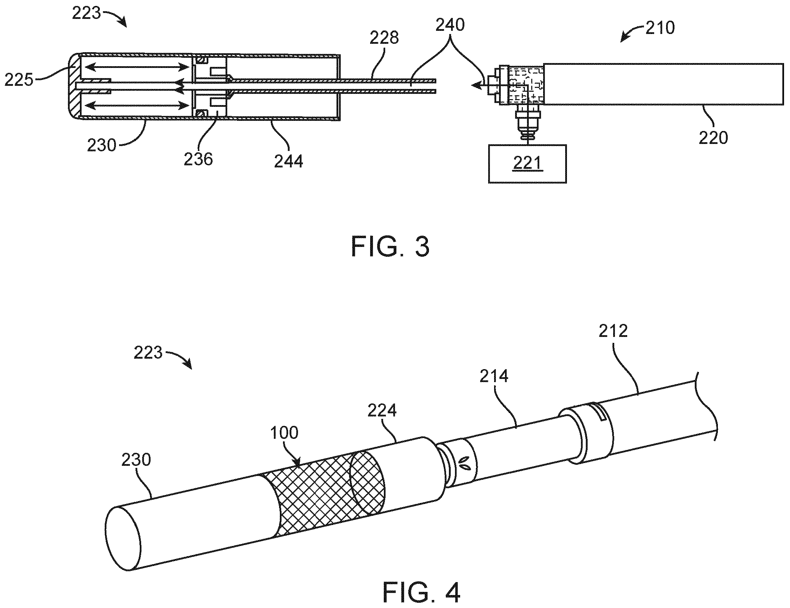

To unsheathe the prosthetic valve 100, the distal capsule 230 can be distally advanced by movement of the shaft 228 via the handle assembly 220 and the proximal capsule 224 can be proximally withdrawn via proximal movement of the inner catheter 234 with the handle assembly 220. Alternatively, unsheathing of the prosthetic valve 100 can be accomplished with a combination of moving both the proximal and distal capsules 224, 230. In one example of the disclosure, movement of the distal capsule 230 is enabled via hydraulics, in any manner known in the art. One fluid path 240, for example, is shown in FIG. 3. In this example, the handle assembly 220 is connected to a fluid source 221. The fluid path 240 extends from the handle assembly 220, through the shaft 228 and to the distal capsule 224. In some embodiments, the distal capsule 224 can include a cavity to receive fluid from the fluid path 240, to correspondingly control the position of the distal capsule 223.

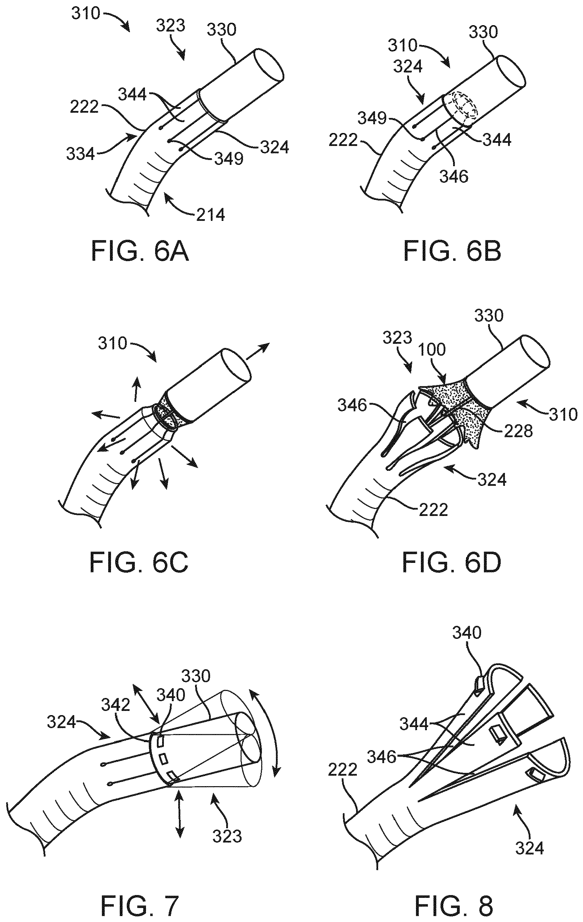

Referring now in addition to FIGS. 6A-9, which illustrate select components of a system having a delivery device 310 and a prosthesis (e.g., implant 100). As indicated with like reference numerals, the delivery device 310 of FIGS. 6A-9 is identically configured and used to that of FIGS. 2A-5C except as explicitly stated. In this example, the delivery device 310 includes a capsule assembly 323 including a proximal capsule 324 and a distal capsule 330, wherein both the proximal and distal capsules 324, 330 are configured to sheathe the implant 100 in the loaded arrangement (FIG. 6A). In various examples of the disclosure, the distal capsule 330 and the proximal capsule 324 overlap in the loaded arrangement (see, FIG. 6B, however, the implant is not shown for ease of illustration). The proximal capsule 324 can be integrally formed with the catheter 222 or can be separately connected to a distal end of the catheter 222. As shown in FIGS. 7-8, in various embodiments, the proximal capsule 324 and the distal capsule 330 each include corresponding features 340, 342 that can be releasably engaged. Not all corresponding features 340, 342 are referenced for ease of illustration. Generally, the proximal and distal capsules 324, 330 are engaged via the corresponding features 340, 342 during delivery in the loaded arrangement. Once the implant 100 is in place at a heart valve, for example, the proximal and distal capsules 324, 330 can be selectively separated by disengaging the corresponding features 340, 342 to unsheathe the implant 100, allowing the implant to expand and deploy from the delivery device 310.

The proximal capsule 324 of FIGS. 6A-9 includes a plurality of slats 344, separated by slits 346 (only a few slats and slits are shown for ease of illustration). In one example, the length of the slit 346 (inherently defining a length of the adjacent slat 344) can be in the range of 1 cm - 10 cm. The number of each of the slits 346 and slats 344 can vary. For example, the number of slits 346 and slats 344 can be in the range of 3 to 12. A proximal end of each slit 346 can optionally include widened portion 349 to provide greater ease for the slats 344 to flex with respect to the catheter 222. In one example, the slats 344 are formed of a shape memory material and are configured such that release of the slats 344 from engagement with the distal capsule 330 allows the stats 344 to transition to a flared arrangement (FIG. 8) in which a distal end of the proximal capsule 324 flares outwardly with respect to the catheter 222. In various embodiments, the degree of flare of the proximal capsule 324 with respect to the catheter 222 is in the range of 0 to 20 degrees. The flared slats 344 transition to collectively form a funnel that can be utilized to recapture the implant 100 within the proximal capsule 324 after partial deployment, as desired. The funnel formed by the proximal capsule 324 can be collapsed for recapture or withdrawal from the patient by distally advancing the outer sheath 212 over the proximal capsule 324, for example.

The corresponding engagement features that selectively interconnect the proximal and distal capsules 324, 330 can take many forms. In the example of FIGS. 6A-9 the corresponding engagement features include tabs 340 and apertures 342. The tabs 340 may be included on the distal end of one or more slats 344 and the apertures 342 can be included on a proximal end of the distal capsule 330. In one example, each tab 340 is made of a resilient or shape memory material. As perhaps best shown in FIG. 8, each tab 340 can extend outwardly from an outer surface of the respective slat 344 and be angled either toward or away from the distal capsule 330. In an alternate embodiment, the tabs 340 can be provided on the distal capsule 330 and the apertures 342 can be provided on the slats 344 or proximal capsule 324. Optionally, the apertures 342 and tabs 340 may be configured such that rotational movement of the proximal capsule 324 with respect to the distal capsule 330 releases the tabs 340 from the apertures 342. In this example, each aperture 342 can include a ramp 348 to guide tabs 340 in and out of engagement depending on the direction of rotation of the proximal capsule 324 with respect to the distal capsule 330. Alternatively, the corresponding engagement features 340, 342 can be configured such that depressing the tabs 340 to collapse the slats 344 (i.e. reduce the flared nature of the proximal capsule 324), disengages the tabs 340 from the apertures 342.

In various embodiments, additional flexibility during steering of the capsule assembly 323 can be provided by including laser cut patterns in the slats 344 as the junction of the proximal capsule 324 and the distal capsule 330 collectively provides a hinge.

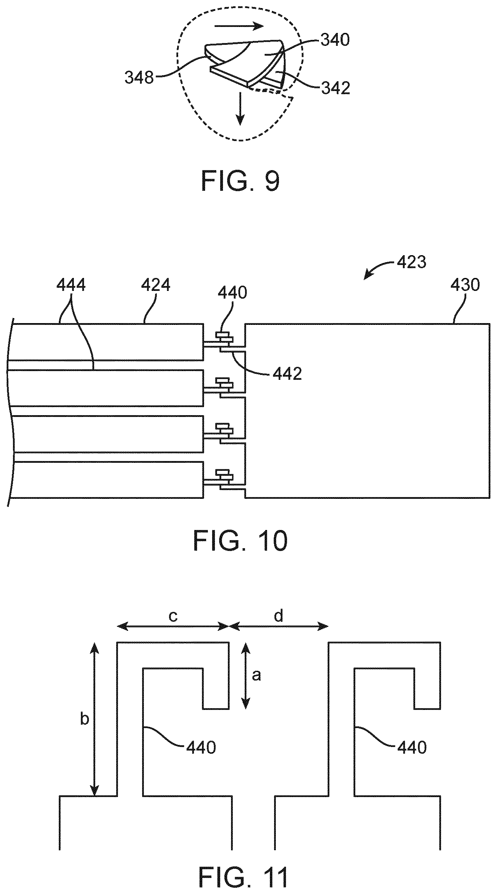

Referring now in addition to FIGS. 10-11, which schematically illustrate an alternate capsule assembly 423 including a proximal capsule 424 and a distal capsule 430 having corresponding engagement features 440, 442. In this example, the proximal capsule 424 and distal capsule 430 each including interlocking engagement features 440, 442. In the example shown, the proximal capsule 424 includes a hook 440 at a distal end of at least one slat 444 and the distal capsule 430 includes a plurality of hooks 442 extending proximally from the proximal end and corresponding to the placement and number of hooks 440 of the proximal capsule 424. Only a few features 440, 442 are referenced for ease of illustration. The proximal capsule hooks 440 are configured to releasably engage the distal capsule hooks 442 to interconnect the capsules 424, 420 to substantially sheathe the implant in the loaded arrangement similar to that of FIG. 5A. In this example, "substantially sheathe" is a state in which the implant is sheathed such that it cannot expand to its natural state. In various embodiments, each hook 440, 442 is L or J-shaped and is made from a shape memory or other metal. As shown in FIG. 11, in one example, each hook 440, 442 can have a dimension "a" that is greater than ½ of the dimension "b" and a dimension "d" that is greater than a dimension "c". Although only hooks 440 are shown in FIG. 11, hooks 442 of the distal capsule can be identically configured. To release the proximal and distal capsules 424, 430 for deployment of the implant 100, the proximal capsule 424 can be distally moved and optionally additionally rotated about a central axis of the proximal capsule 424. Once the distal capsule 430 is disengaged from the proximal capsule 424, the distal capsule 430 can be distally advanced by distally advancing the shaft 228 to free the implant 100 from the capsule assembly 423 for full or partial deployment (see also FIG. 2B, 5A, 5C). Alternatively, or in addition, the proximal capsule 424 can be proximally withdrawn by proximally pulling the catheter 222 to unsheathe the implant 100. The proximal and distal capsules 424, 430 can be incorporated into any of the devices disclosed herein and used in an identical manner except as explicitly stated.

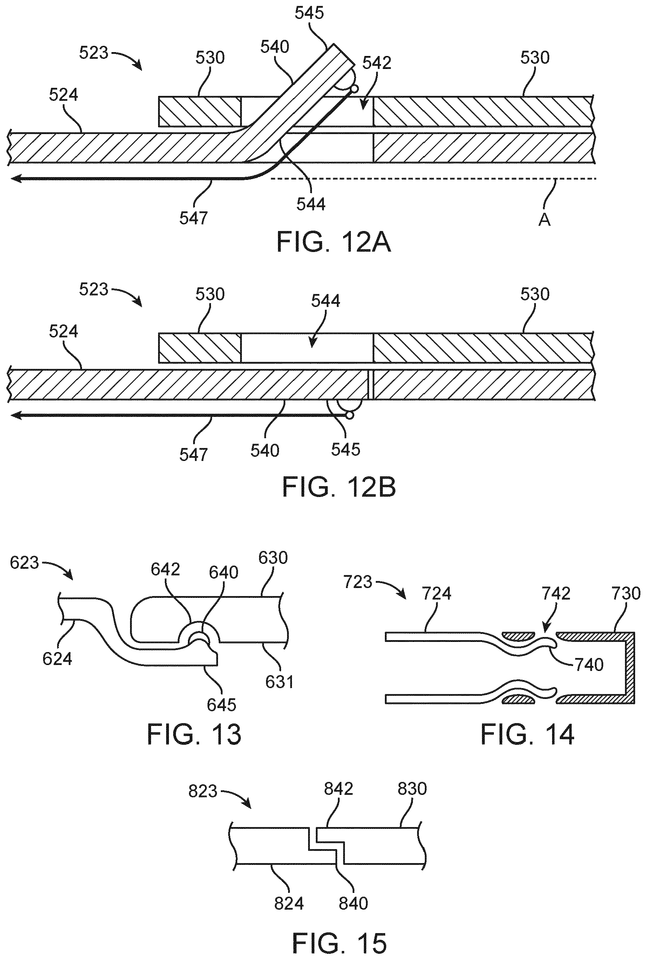

Referring now in addition to FIGS. 12A-12B, which schematically illustrate an alternate capsule assembly 523 having a proximal capsule 524 and a distal capsule 530. The proximal and distal capsules 524, 530 include corresponding engagement features 540, 542. In this example, the proximal capsule 524 includes plurality of slats 544 (similar to what is shown and described with respect to FIG. 6A, for example), each slat 544 having a distal end 545 formed of a shape memory metal or other resilient, biased material that is configured to be curved away from a central axis A of the proximal capsule 524. The engagement features 542 of the distal capsule 530 include apertures 542 in which each of the distal ends 545 of slats 544 can be received to interlock the proximal and distal capsules 524, 530. To release the proximal capsule 524 from the distal capsule 530, a pull wire 547 is secured to each distal end 545 and extends proximally to the handle assembly. Proximal tension of the pull wire 547 pulls the respective slat 544 against its biased, curved arrangement of FIG. 12A to be straightened as shown in FIG. 12B. This straightening pulls the distal end 545 from the aperture 542 so that the proximal capsule 524 can be proximally withdrawn to at least partially unsheathe the implant positioned within the capsule assembly 523 (see also, FIGS. 5B and 5C). The capsule assembly 523 can be incorporated into any delivery device disclosed herein and used in an identical manner except as expressly stated.

Referring now to FIG. 13, which schematically illustrates an alternate capsule assembly 623 including a proximal capsule 624 and a distal capsule 630 having corresponding engagement features 640, 642. In this example, the proximal capsule 624 includes a distal end 645 having a protrusion 640 that is configured to selectively engage a groove 642 formed in an inner surface 631 of the distal capsule 630. The proximal capsule 624 may or may not have slats, similar to slats 344 disclosed above. In such embodiments having slats, the protrusion 640 would be located on a distal end of one or more of the slats. The capsule assembly 623 can be incorporated into any delivery device disclosed herein and used in an identical manner except as expressly stated.

Referring now in addition to FIG. 14, which schematically illustrates an alternate capsule assembly 723 having a proximal capsule 724 and a distal capsule 730 having corresponding engagement features 740, 742. In this example, the proximal capsule 724 includes at least one resilient spring leaf 740 that is configured to selectively engage a corresponding aperture 742 formed the distal capsule 730. The proximal capsule 724 may or may not have slats, similar to slats 344 disclosed above. Separation of the corresponding engagement features 740, 742 is accomplished via axial transition of the proximal capsule 724. The capsule assembly 723 can be incorporated into any delivery device disclosed herein and used in an identical manner except as expressly stated.

Referring now in addition to FIG. 15, which schematically illustrates an alternate capsule assembly 823 having a proximal capsule 824 and a distal capsule 830 having corresponding engagement features 840, 842. In this example, the proximal capsule 824 includes a radial lip 840 at its distal end that is configured to selectively engage a corresponding radial lip 842 formed at a proximal end of the distal capsule 830. Separation of the corresponding engagement features 840, 842 is accomplished by axial translation of the distal capsule 830 away from the proximal capsule 824. The capsule assembly 823 can be incorporated into any delivery device disclosed herein and used in an identical manner except as expressly stated.

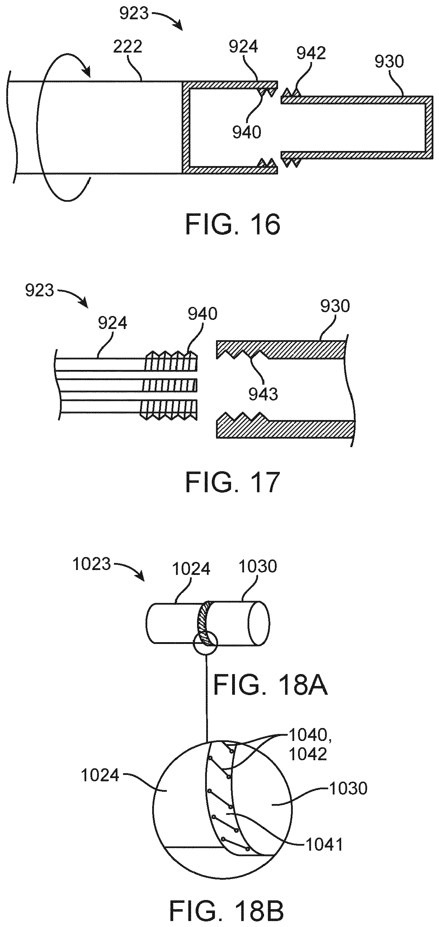

Referring now in addition to FIG. 16, which schematically illustrates an alternate capsule assembly 923 including a proximal capsule 924 and a distal capsule 930 having corresponding engagement features 940, 942. In this example, the proximal capsule 924 includes a threaded interface 940 at its distal end that is configured to selectively engage a corresponding threaded surface 942 formed at a proximal end of the distal capsule 930. Separation of the corresponding engagement features 940, 942 is accomplished by rotating one of the corresponding engagement features 940, 942 about a central axis of the capsule assembly 923 to release the threads 940, 942. In the example, of FIG. 16, the distal capsule 930 can be positioned within the proximal capsule 924. In the example of FIG. 17, the proximal capsule 924 can be positioned within the distal capsule 930. The capsule assembly 923 can be incorporated into any delivery device disclosed herein and used in an identical manner except as expressly stated.

Referring now in addition to FIGS. 18A-18B, which schematically illustrates an alternate capsule assembly 1023 including a proximal capsule 1024 and a distal capsule 1030 having corresponding engagement features 1040, 1042 (only some of which are referenced for ease of illustration). In this example, the proximal capsule 1024 includes a plurality of apertures 1040 (generally referenced) that are aligned with a plurality of corresponding apertures 1042 formed in the distal capsule 1030. An elongated member 1041 is threaded through the apertures 1040, 1042 to secure the proximal capsule to the distal capsule 1030. The elongated member 1041 can be a suture, wire, thread, filament or the like. Separation of the corresponding engagement features 1040, 1042 can optionally be accomplished by retracting the elongated member 1041. The capsule assembly 1023 can be incorporated into any delivery device disclosed herein and used in an identical manner except as expressly stated.

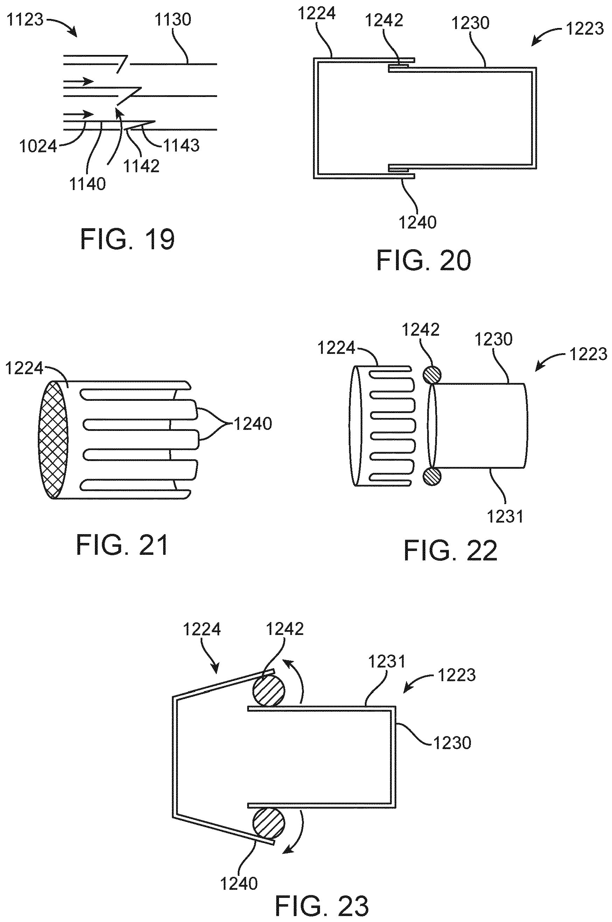

Referring now in addition to FIG. 19, which schematically illustrates an alternate capsule assembly 1123 having a proximal capsule 1124 and a distal capsule 1130 having corresponding engagement features 1140, 1142. Only one of each feature 1140, 1142 is referenced for ease of illustration. In this example, the proximal capsule 1124 includes a plurality of slats 1140, each having a tab 1143 that are configured to selectively engage slots 1142 formed within the distal capsule 1130. Separation of the corresponding engagement features 1140, 1142 is accomplished by compressing the proximal capsule 1024 towards the distal capsule 1030. This compression causes the distal portion of the "slot" or "aperture" 1142 to push against the tab 1143. By pushing on the tab 1143, the slat 1140 will act like a hinge and pivot radially outward towards an inner wall of the proximal capsule 1124. Once pushed far enough, the tab 1143 will be flush against the wall of the proximal capsule 1124 and will no longer be engaged with the slot/aperture 1142. The proximal or distal capsule 1124, 1130 is rotated (so the slat 1140 can no longer interact with the slot 1142 once the proximal capsule 1124 is moved proximally). Then, the proximal capsule 1124 can be pulled away from the distal capsule 1130. The capsule assembly 1123 can be incorporated into any delivery device disclosed herein and used in an identical manner except as expressly stated.

Referring now in addition to FIGS. 20-23, which schematically illustrate an alternate capsule assembly 1223 having a proximal capsule 1224 and a distal capsule 1230 having corresponding engagement features 1240, 1242. In this example, the proximal capsule 1224 (shown disconnected from catheter 222 or the like) includes a plurality of slats 1240 that are configured to frictionally engage a deflated balloon 1242 secured to an outer surface 1231 of the distal capsule 1230. In one example, the balloon spans 360 degrees of the distal capsule 1230. To selectively separate the proximal capsule 1224 from the distal capsule 1230, the balloon is inflated pushing the slats 1240 to bow away from the distal capsule 1230 so that the proximal capsule 1224 can be disengaged and proximally withdrawn from the distal capsule 1230. Alternatively, the distal capsule 1230 could include the slats and the balloon could be secured to the proximal capsule. The capsule assembly 1223 can be incorporated into any delivery device disclosed herein and used in an identical manner except as expressly stated.

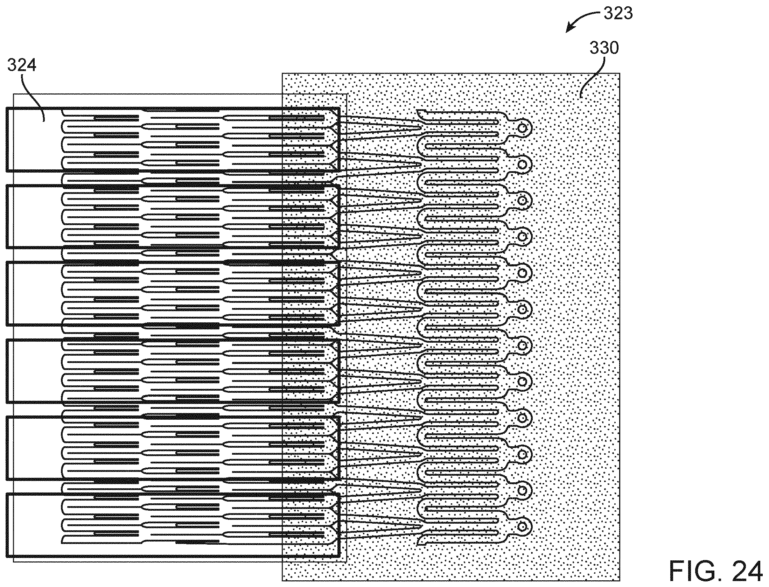

In the non-limiting example of FIG. 24, any distal capsule of the disclosure can be sized to cover 14 mm of a distal end of the stent of the implant in the compressed arrangement. In one example, referencing the capsule assembly of FIGS. 6A-8, the proximal and distal capsules 324, 330 are configured to overlap an amount in the range of 1 mm to 20 mm in the loaded arrangement. In another example, the proximal and distal capsules 324, 330 are configured to overlap an amount in the range of 2 mm to 5 mm in the loaded arrangement. In various embodiments, the proximal capsule 324 would be configured to cover the remaining stent 13-16 mm and brim 170 being about 10 mm (see also, FIG. 1B) in the loaded arrangement. Any of the other capsule assemblies of the disclosure can be similarly configured.

In further envisioned embodiments, capsule assemblies of the disclosure can include a proximal capsule and a distal capsule that are releasably connected to at least partially deploy an implant with electromagnet attachment, dissolvable glue, or hook and loop fasteners. In addition, it is envisioned that the proximal and distal capsules could be secured via a fuse that can be melted to release the connection to provide a releasable connection between the proximal and distal capsules.

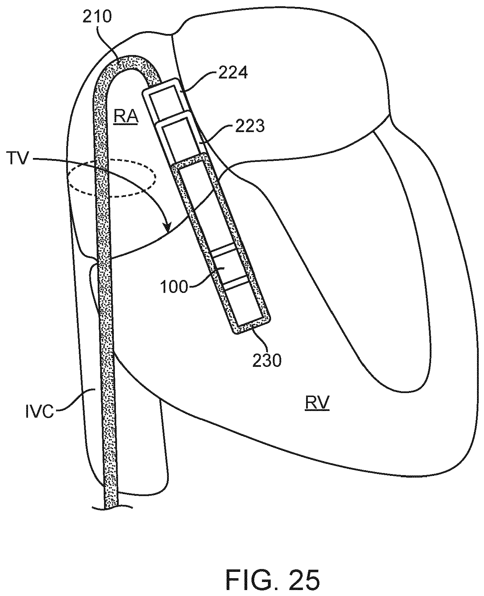

In various embodiments, any delivery device of the disclosure having any capsule assembly of the disclosure can be used as schematically depicted in FIG. 25. The delivery device 210 is referenced as one non-limiting example and it is to be understood that other delivery devices disclosed herein can be delivered in a similar manner and similarly used to deploy an implant at a heart valve. The delivery device 210 can be provided in the loaded arrangement having the implant 100 in the compressed, loaded arrangement having the proximal and distal capsules 224, 230 engaged and fully covering the implant 100. Optionally, the outer sheath (not shown) can be positioned over the capsule assembly 223. In other examples, the outer sheath is positioned proximal with respect to the capsule assembly 223 during delivery to reduce device profile, particularly at the distal end of the delivery device. The loaded implant 100 can be directed through a patient's femoral view to the inferior vena cava IVC and into the right atrium RA. In the right atrium RA, the engagement feature(s) (not visible) are disengaged to correspondingly disengage the proximal capsule 224 from the distal capsule 230. In some embodiments, the inner member can be proximally withdrawn as the slats of the proximal capsule flare outwardly after being freed from the distal capsule. It is noted that the outward expansion of the slats lowers the force needed to retract the proximal capsule. At this stage, the implant is at least partially exposed from the capsule assembly, allowing for greater visualization to aid with imaging and implant placement. Then, the implant can be positioned within a tricuspid valve TV. In some examples, the distal capsule 230 is distally advanced within right ventricle RV to unsheathe the implant 100 while the proximal capsule 224 remains within the right atrium RA. Some methods include recapturing the implant recapture at least partially within the capsule assembly prior to full deployment and release of the implant from the delivery device. Partial deployment of the implant from the proximal and distal capsules can revert to a more fully sheathed or recaptured implant for repositioning. Once the desired implant positioning is achieved, the capsule assembly can be transitioned to fully unsheathe the implant, allowing the implant to expand and release from the delivery device. Then the capsule assembly can be proximally withdrawn and removed from the patient in the same manner as it was delivered.

Devices and methods of the disclosure are beneficial in that they can be configured to reduce the travel depth of the delivery device into the right ventricle, which can increase the patient population that can receive larger valves. The present inventors additionally believe that embodiments of the disclosure require lower deployment forces. Additionally, the juncture between the distal and proximal capsules has been found to provide a useful echogenic landmark during delivery of the implant. Additional benefits will be apparent to one of skill in the art in view of the present disclosure.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

## Claims

1. A delivery device (210) comprising:
a handle assembly (220);
an inner catheter (222) connected to the handle assembly and having a distal portion configured to receive an implant (100);
an outer catheter (212) connected to the handle assembly and coaxially positioned over the inner catheter; and
a capsule assembly (223) including:
a proximal capsule (224) secured to a distal end of the inner catheter; the proximal capsule defining a plurality of slats (344) separated by a plurality of slits (346), and
a distal capsule (230) connected to a capsule shaft (228) that extends within the inner catheter, the capsule shaft interconnecting the distal capsule to the handle assembly; wherein the capsule assembly includes engagement features (340, 342, 440, 442, 540, 542, 640, 642, 740, 742, 840, 842, 940, 942, 1040, 1042, 1140, 1142, 1240, 1242) to releasably secure the distal capsule to the proximal capsule.

2. The delivery device of claim 1, wherein the distal capsule is configured to be positioned partially over the proximal capsule when the engagement features are engaged.

3. The delivery device of claim 1 or 2, wherein the engagement features include apertures (342, 1142) and tabs (340, 1143).

4. The delivery device of claim 3, wherein each aperture includes a ramp (348).

5. The delivery device of claim 1 or 2, wherein the engagement features include interlocking tabs.

6. The delivery device of claim 5, wherein the interlocking tabs are each hook shaped (440, 442).

7. The delivery device of according to any of the preceding claims, wherein the proximal capsule has an uncompressed arrangement having a flared distal end as compared to a compressed arrangement when the distal end is engaged with the distal capsule.

8. The delivery device according to any of the preceding claims, further comprising a pull wire (547) secured to a distal end (545) of each slat (544).

9. The delivery device according to any of the claims 1 to 7, wherein a pull wire (547) is secured to each slat (544).

## Patentansprüche

1. Zuführvorrichtung (210), umfassend:
eine Griffbaugruppe (220),
einen Innenkatheter (222), der mit der Griffbaugruppe verbunden ist und einen distalen Abschnitt aufweist, der zur Aufnahme eines Implantats (100) ausgestaltet ist,
einen Außenkatheter (212), der mit der Griffbaugruppe verbunden und koaxial über dem Innenkatheter positioniert ist, und
eine Kapselbaugruppe (223), die Folgendes aufweist:
eine proximale Kapsel (224), die an einem distalen Ende des Innenkatheters befestigt ist, wobei die proximale Kapsel eine Vielzahl von Lamellen (344) definiert, die durch eine Vielzahl von Schlitzen (346) getrennt sind, und
eine distale Kapsel (230), die mit einem Kapselschaft (228) verbunden ist, der sich in dem Innenkatheter erstreckt, wobei der Kapselschaft die distale Kapsel mit der Griffbaugruppe verbindet, wobei die Kapselbaugruppe Eingriffsmerkmale (340, 342, 440, 442, 540, 542, 640, 642, 740, 742, 840, 842, 940, 942, 1040, 1042, 1140, 1142, 1240, 1242) aufweist, um die distale Kapsel lösbar an der proximalen Kapsel zu befestigen.

2. Zuführvorrichtung nach Anspruch 1, wobei die distale Kapsel dazu ausgestaltet ist, teilweise über der proximalen Kapsel positioniert zu sein, wenn die Eingriffsmerkmale in Eingriff stehen.

3. Zuführvorrichtung nach Anspruch 1 oder 2, wobei die Eingriffsmerkmale Öffnungen (342, 1142) und Laschen (340, 1143) aufweisen.

4. Zuführvorrichtung nach Anspruch 3, wobei jede Öffnung eine Rampe (348) aufweist.

5. Zuführvorrichtung nach Anspruch 1 oder 2, wobei die Eingriffsmerkmale ineinandergreifende Laschen aufweisen.

6. Zuführvorrichtung nach Anspruch 5, wobei die Verriegelungslaschen jeweils hakenförmig (440, 442) sind.

7. Zuführvorrichtung nach einem der vorhergehenden Ansprüche, wobei die proximale Kapsel eine nicht zusammengedrückte Anordnung mit einem aufgeweiteten distalen Ende im Vergleich zu einer zusammengedrückten Anordnung, wenn das distale Ende mit der distalen Kapsel in Eingriff steht, aufweist.

8. Zuführvorrichtung nach einem der vorhergehenden Ansprüche, ferner umfassend einen Zugdraht (547), der an einem distalen Ende (545) jeder Lamelle (544) befestigt ist.

9. Zuführvorrichtung nach einem der Ansprüche 1 bis 7, wobei ein Zugdraht (547) an jeder Lamelle (544) befestigt ist.

## Revendications

1. Dispositif d'administration (210) comprenant :
un ensemble poignée (220) ;
un cathéter interne (222) relié à l'ensemble poignée et ayant une partie distale configurée pour recevoir un implant (100) ;
un cathéter externe (212) relié à l'ensemble poignée et positionné coaxialement sur le cathéter interne ; et
un ensemble capsule (223) comprenant :
une capsule proximale (224) fixée à une extrémité distale du cathéter interne, la capsule proximale définissant une pluralité de lattes (344) séparées par une pluralité de fentes (346), et
une capsule distale (230) reliée à une tige de capsule (228) qui s'étend à l'intérieur du cathéter interne, la tige de capsule interconnectant la capsule distale à l'ensemble poignée ; l'ensemble capsule comprenant des éléments de mise en prise (340, 342, 440, 442, 540, 542, 640, 642, 740, 742, 840, 842, 940, 942, 1040, 1042, 1140, 1142, 1240, 1242) pour fixer de façon libérable la capsule distale à la capsule proximale.

2. Dispositif d'administration selon la revendication 1, la capsule distale étant configurée pour être positionnée partiellement sur la capsule proximale lorsque les éléments de mise en prise sont en prise.

3. Dispositif d'administration selon la revendication 1 ou 2, les éléments de mise en prise comprenant des ouvertures (342, 1142) et des pattes (340, 1143).

4. Dispositif d'administration selon la revendication 3, chaque ouverture comprenant une rampe (348).

5. Dispositif d'administration selon la revendication 1 ou 2, les éléments de mise en prise comprenant des pattes de verrouillage réciproque.

6. Dispositif d'administration selon la revendication 5, les pattes de verrouillage réciproque ayant chacune une forme de crochet (440, 442).

7. Dispositif d'administration selon l'une quelconque des revendications précédentes, la capsule proximale ayant un agencement non comprimé ayant une extrémité distale évasée par rapport à un agencement comprimé lorsque l'extrémité distale est en prise avec la capsule distale.

8. Dispositif d'administration selon l'une quelconque des revendications précédentes, comprenant en outre un fil de traction (547) fixé à une extrémité distale (545) de chaque latte (544).

9. Dispositif d'administration selon l'une quelconque des revendications 1 à 7, un fil de traction (547) étant fixé à chaque latte (544).
